Europäisches Patentamt

European Patent Office

Office européen des brevets

(11) Veröffentlichungsnummer: **0 217 413**
A2

(12) # EUROPÄISCHE PATENTANMELDUNG

(21) Anmeldenummer: 86113896.8

(51) Int.Cl.⁴: **A 61 K 7/48**

(22) Anmeldetag: 03.10.86

(30) Priorität: 03.10.85 DE 3535377

(43) Veröffentlichungstag der Anmeldung:
08.04.87 Patentblatt 87/15

(84) Benannte Vertragsstaaten:
AT BE CH DE ES FR GB IT LI LU NL SE

(71) Anmelder: Chemisches Laboratorium Dr. Kurt Richter
GmbH
Bennigsenstrasse 25
D-1000 Berlin 41(DE)

(72) Erfinder: Petersen, Rolf-Dieter, Dr. rer. nat. Dipl.-Chem.
Deisterpfad 36
D-1000 Berlin 37(DE)

(72) Erfinder: Borchert, Günter, Dipl.-Ing.
Waldsassener Strasse 63 d
D-1000 Berlin 48(DE)

(74) Vertreter: Mitscherlich, Hans, Dipl.-Ing. et al,
Patentanwälte Dipl.-Ing. H. Mitscherlich Dipl.-Ing. K.
Gunschmann Dipl.-Ing. Dr.rer.nat. W. Körber Dipl.-Ing. J.
Schmidt-Evers Dipl.-Ing. W. Melzer Steinsdorfstrasse 10
D-8000 München 22(DE)

(54) **Kosmetische Mittel.**

(57) Kosmetische Mittel, die einen die Kollagenase-Aktivität der Haut fördernden Wirkstoff enthalten, um der im Verlauf der Hautalterung, aber auch durch übermäßige Licht- und Sonnenexposition der Haut nachlassenden Kollagenase-Aktivität der Haut entgegenzuwirken. Der Wirkstoff besteht aus einer aus Blutserum gewonnenen Proteinfraktion oder aus Polysaccharidsulfaten wie Heparin oder Dextransulfat. Die kosmetischen Mittel können ferner natives lösliches Kollagen (Tropokollagen) als weiteren Wirkstoff enthalten.

EP 0 217 413 A2

## Kosmetische Mittel          0217413

Es ist bekannt, daß im Verlauf der Hautalterung der Vernetzungsgrad des Kollagens (intermolekulare kovalente Bindungen zwischen Tropokollagenmolekülen), d.h. der unlösliche Kollagenanteil zunimmt, was eine verminderte Hautelastizität zur Folge hat. Aus der DE-PS 2o 64 6o4 C3 sind Hautpflegemittel bekannt, deren Ziel es ist, durch Verwendung von nativem löslichen Kollagen (Tropokollagen) den löslichen, d.h. unvernetzten Anteil des Kollagens in der Haut zu erhöhen. Dadurch wird das sich altersbedingt verschiebende Verhältnis von löslichem zu unlöslichem Kollagen zugunsten der löslichen Fraktion verbessert und der Elastizitätsverlust der Haut verlangsamt.

Die Hautfibroblasten synthetisieren bekanntlich neben Kollagen auch andere Makromoleküle des Bindegewebes (z.B. Elastin) und sind darüber hinaus der wichtigste Entstehungsort der Hautkollagenase, einem Enzym, das den Kollagenabbau einleitet. Im Fibroblast wird zunächst ein Zymogen, d.h. eine inaktive Vorstufe dieses kollagenspezifischen Enzyms, die sogenannte Prokollagenase, synthetisiert und in den Extrazellularraum ausgeschleust. Durch komplexe Steuervorgänge erfolgt dann je nach Bedarf die proteolytische Aktivierung (z.B. durch Trypsin) zur eigentlichen Kollagenase. Bei diesen Steuerungsvorgängen sind auch Kollagenase-Inhibitoren beteiligt, die durch Bildung eines Enzym-Inhibitor-Komplexes die proteolytische Wirkung aufheben. Inhibitoren sind z.B. $\alpha^2$-Makroglobulin, isoliert aus dem Serum, oder Lysozyme oder Protamine. Während Struktur und Angriffspunkt der Kollagenase am fibrillären Kollagen bekannt sind, sind die spezifischen Prozesse, die den Kollagenabbau durch Aktivierung bzw. Inhibierung des Enzyms in vivo steuern, weitgehend unbekannt.

Der Kollagenstoffwechsel, d.h. die Neusynthese im Fibroblast und die Metabolisierung, eingeleitet durch Kollagenase, unterliegt derartigen Steuermechanismen. Es ist jedoch festzustellen, daß im Verlauf der Alterung, aber auch durch übermäßige Licht- und Sonnenexposition der Haut, der Vernetzungsgrad, d.h. der Anteil an unlöslichem Kollagen zunimmt, was unter anderem auf ein Nachlassen der Kollagenase-Aktivität zurückzuführen ist.

Der vorliegenden Erfindung liegt nun die Aufgabe zugrunde, die nachlassende Kollagenase-Aktivität der Haut im physiologisch erwünschten Maß zu erhöhen, um den Anteil an unlöslichem Kollagen durch erhöhten Abbau zu verringern und den Kollagenstoffwechsel insgesamt zu verbessern.

Die gestellte Aufgabe wird erfindungsgemäß dadurch gelöst, daß durch ein kosmetisches Mittel der Haut ein die Kollagenase-Aktivität steigernder Wirkstoff lokal zugeführt wird. Es hat sich überraschenderweise gezeigt, daß es gelingt, durch die topische Applikation die Kollagenase-Aktivität zu erhöhen, was zu einer Verminderung des quervernetzten, d.h. unlöslichen Anteils des Hautkollagens führt.

Als ein die Kollagenase-Aktivität fördernder Wirkstoff eignet sich insbesondere eine aus Blutserum gewonnene Proteinfraktion, die bis ca. 80° C thermostabil ist und ein Molekulargewicht von ca. 2o.ooo aufweist.

Die Herstellung dieses erfindungswesentlichen Wirkstoffes auf Basis einer Proteinfraktion erfolgt nach bekannten Fraktionierungsverfahren, wie sie sich der Serum- und Plasmagewinnung anschließen. Dazu wird Blutserum in einer Konzentration von 5 bis 1o % in 0,05 M Tris-Puffer, pH 7,5, gelöst und einer Salzfraktionierung unterworfen. Diese erfolgt durch Zusatz von maximal 3o % Ammoniumsulfat. Die ausgesalzten Proteinbestandteile des Serums (u.a. Enzym-Inhibitoren) werden abgetrennt. Zur Beseitigung des Ammoniumsulfats schließt sich eine Dialyse an. Die Trenngrenze der verwendeten semipermeablen Membran liegt vorzugsweise bei einem Molekulargewicht von 3.5oo. Das Dialysat befindet sich nach Abschluß des Verfahrens in einem 0,05 M Trispuffer, pH 7,5, mit 0,15 M NaCl. Durch ein sich anschließendes kurzzeitiges Temperieren auf max. 80° C - der Kollagenase-Aktivator ist in diesem Temperaturbereich stabil - werden weitere störende Seruminhaltsstoffe denaturiert und zur Koagulation gebracht. Die endgültige Klärung erfolgt durch Zentrifugation. Der Kollagenase-Aktivator liegt in gelöster Form in dem klaren Überstand vor.

Der die Kollagenase aktivierende Wirkstoff aus Blutserum ist ein Protein mit einem Molekulargewicht von ca. 2o.ooo, das gegen einen enzymatischen Abbau, z.B. durch Trypsin, Chemotrypsin und Thermolysin resistent ist, jedoch durch die unspezifische Protease Pronase abgebaut werden kann. Dieses Aktivatorprotein besitzt selbst keine Enzymaktivität, greift also das Kollagen nicht an. Es beeinflußt darüber hinaus nicht die Spezifität der Kollagenase, da die Spaltprodukte des mit Kollagenase umgesetzten fibrillären Kollagens mit und ohne Aktivatorzusatz unverändert bleiben.

Die Kollagenase aktivierende Wirkung des Proteins konnte in einer in vivo Testreihe an Ratten wie folgt nachgewiesen werden:

Auf das gesamte Testareal rasierter Rückenhaut von Ratten (3 Gruppen zu je 6 Tieren) wurden die folgenden Testcremes aufgetragen:

1. O/W-Creme mit Kollagenase-Aktivator aus Blutserum

2. O/W-Kontrollcreme mit Puffer

(3.) Eine Kontrollgruppe blieb unbehandelt.

Um die der natürlichen Alterung gemäßen Veränderungen des Bindegewebes künstlich zu beschleunigen, wurde jeweils eine Hälfte des Testareals mit UV-Licht bestrahlt. Die andere Hälfte blieb während der Bestrahlung abgedeckt. Innerhalb der Testdauer von 14 Tagen wurden den Tieren täglich vor und nach Bestrahlung die jeweiligen Testcremes aufgetragen. Die Bestrahlung erfolgte einmal täglich.

Nach Tötung der Tiere unter Ethernarkose wurde aus den jeweiligen Testarealen nach entsprechender Aufarbeitung der Anteil des löslichen Kollagens sowie das Gesamtkollagen über die Hydroxyprolinmenge pro g Hautfrischgewicht bestimmt. Nach statistischer Auswertung des Zahlenmaterials zeigten sich folgende Ergebnisse:

Die Testgruppe, die mit Kollagenase-Aktivator-haltiger Creme behandelt wurde, zeigte gegenüber den Kontrollgruppen eine signifikante Abnahme des Gehaltes an unlöslichem Kollagen, ermittelt aus dem Gesamtkollagengehalt und dem löslichen Kollagenanteil. Dieser erhöhte Abbau vernetzten Kollagens ist auf die wirkstoffbedingte Förderung der Kollagenase-Aktivität zurückzuführen.

Als weitere die Kollagenase-Aktivität fördernde Wirkstoffe eignen sich Polysaccharidsulfate wie Heparin oder Dextransulfat. - ι

Um neben der durch den Kollagenase-Aktivator geförderten Reduzierung des Quervernetzungsgrades gleichzeitig eine Steigerung des Anteils der löslichen Kollagenfraktion zu bewirken, setzt man zweckmäßigerweise dem kosmetischen Mittel noch natives lösliches Kollagen (Tropokollagen) zu.

Die nachfolgenden Rezepturen sind Ausführungsbeispiele der Erfindung:

### Beispiel 1

Creme, O/W mit Kollagenase-Aktivator

a) Cetylstearylalkohol 9,0 %
   Natrium-Cetylstearylsulfat 1,0 %
   Ölsäuredecylester 5,0 %
   Wollwachs 2,0 %
   Capryl/Caprinsäuretriglycerid 1C,0 %
   Paraffinöl, dickflüssig 5,0 %
   Konservierungsmittel q.s.

b) Wasser, destilliert 58,0 %
   Konservierungsmittel q.s.
   70%ige Lösung von Sorbit 5,0 %

c) Kollagenase-Aktivator 5,0 %

Herstellung:

a) schmelzen und auf ca. 70° C bringen;
b) auf ca. 70° C erwärmen und in a) einrühren.
Weiterrühren, bis die Creme auf ca. 30° C abgekühlt ist,
c) einrühren.

Beispiel 2

Lotion mit Kollagenase-Aktivator

Wasser, destilliert            92,0 %
Konservierungsmittel           q.s.
1,2-Propylenglykol             3,0 %
Kollagenase-Aktivator          5,0 %

Herstellung:
Der Reihe nach mischen.

Beispiel 3

Gel mit Kollagenase-Aktivator

a) Wasser, destilliert                      65,30 %
   Konservierungsmittel                     q.s.
   Carbopol 94o (Polyacrylsäure)            0,50 %

b) Polyoxyethylen (2o) sorbitantrioleat     0,30 %
   Sorbitanmonooleat                        0,15 %
   Capryl/Caprinsäuretriglycerid            2,50 %

c) Wasser, destilliert                      25,75 %
   Konservierungsmittel                     q.s.
   Triethanolamin                           0,50 %

d) Kollagenase-Aktivator                    5,00 %

Herstellung:

a) Carbopol 94o unter schnellem Rühren dispergieren;
b) mischen und in a) einrühren,
c) mischen und einrühren,
d) einrühren.

**Beispiel 4**

Creme, O/W mit Kollagenase-Aktivator
und nativem löslichem Kollagen (Tropokollagen)

a) Cetylstearylalkohol 9,0 %
   Natrium-Cetylstearylsulfat 1,0 %
   Ölsäuredecylester 5,0 %
   Wollwachs 2,0 %
   Capryl/Caprinsäuretriglycerid 10,0 %
   Paraffinöl, dickflüssig 5,0 %
   Konservierungsmittel q.s.

b) Wasser, destilliert 48,0 %
   Konservierungsmittel q.s.
   70%ige Lösung von Sorbit 5,0 %

c) Kollagenase-Aktivator 5,0 %

d) Natives lösliches Kollagen
   (Tropokollagen) 10,0 %

Herstellung:

a) schmelzen und auf ca. 70° C bringen;
b) auf ca. 70° C erwärmen und in a) einrühren.
Weiterrühren, bis die Creme auf ca. 30° C abgekühlt ist,
c) und d) einrühren.

PATENTANWÄLTE
Dipl.-Ing. H. MITSCHERLICH
Dipl.-Ing. K. GUNSCHMANN
Dipl.-Ing. Dr.rer.nat. W. KÖRBER
Dipl.-Ing. J. SCHMIDT-EVERS
Dipl.-Ing. W. MELZER
EUROPEAN PATENT ATTORNEYS

Telefon (069) 29 66 84-86
Telex 523 155 mitsh d
Telegramme Patentpaap
Telecopier (089) 22 68 34
Psch-Kto. Mchn. 195 75-803
EPA-Kto 28 000 206

Steinsdorfstraße 10
D-8000 München 22

0217413

03.10.1986

Chemisches Laboratorium
Dr. Kurt Richter GmbH

Bennigsenstraße 25

1ooo Berlin 41

## Ansprüche

1. Kosmetische Mittel, dadurch gekennzeichnet, daß sie einen die Kollagenase-Aktivität der Haut fördernden Wirkstoff enthalten.

2. Kosmetische Mittel nach Anspruch 1, dadurch gekennzeichnet, daß der Wirkstoff aus einer aus Blutserum gewonnenen, bis ca. 8o° C thermostabilen Protein-fraktion mit einem Molekulargewicht von ca. 2o.ooo besteht.

3. Kosmetische Mittel nach Anspruch 1, dadurch gekennzeichnet, daß der die Kollagenase-Aktivität fördernde Wirkstoff aus Polysaccharidsulfaten wie Heparin oder Dextransulfat besteht.

4. Kosmetische Mittel nach den Ansprüchen 1 bis 3, gekennzeichnet durch einen Gehalt an nativem löslichem Kollagen (Tropekollagen) als weiteren Wirkstoff.